# EUROPÄISCHE PATENTSCHRIFT

(11) **EP 0 513 071 B1**
(45) Veröffentlichungstag und Bekanntmachung des Hinweises auf die Patenterteilung: **24.08.1994**
(21) Anmeldenummer: 91902952.0
(22) Anmeldetag: 31.01.1991
(51) Int. Cl.: A61M 1/36

(54) **REPERFUSIONSVORRICHTUNG**
REPERFUSION DEVICE
DISPOSITIF DE REPERFUSION

(30) Priorität: 06.02.1990 DE 4003425
(43) Veröffentlichungstag der Anmeldung: 19.11.1992
(73) Patentinhaber: Beyersdorf, Friedhelm, D-65207 Wiesbaden (DE)
(72) Erfinder: Beyersdorf, Friedhelm, D-65207 Wiesbaden (DE)
(74) Vertreter: Vetter, Ewald Otto, Dipl.-Ing.
(86) Internationale Anmeldenummer: EP9100179
(87) Internationale Veröffentlichungsnummer: WO9112036

(56) Entgegenhaltungen:
- EP-A- 0 080 436
- EP-A- 0 192 575
- EP-A- 0 357 338
- FR-A- 2 648 714
- US-A- 4 648 384

## Beschreibung

Die Erfindung betrifft eine Reperfusionsvorrichtung für die Reperfusion von Blutgefäßen nach dem Oberbegriff des Patentanspruchs 1.

Eine solche Reperfusionsvorrichtung ist aus der DE-C-38 20 840 bekannt. Es wird zwar erwähnt, daß der Perfusionsdruck geregelt werden muß, es ist aber keine Druckmessung an der Katheterspitze vorgesehen.

Zur Beseitigung von Gefäßverschlüssen sind verschiedene Methoden bekannt, beispielsweise die chirurgische Revaskularisation, die Dilatation des verengten Gefäßes mit einem Ballonkatheter oder die Thrombolyse, bei der eine Auflösung des Thrombus durch intraarteriell eingebrachte Medikamente erfolgt. Trotz erfolgreicher Wiederherstellung der Blutstrombahn sind Morbidität und Mortalität dieses Eingriffes relativ hoch. Die Hauptursache hierfür ist der Reperfusionsschaden, d.h. eine Gewebeschädigung, die eintritt, wenn das Blut mit dem vom Herzen erzeugten vollen Arteriendruck wieder durch die behandelte Arterie strömt. DE 38 20 840 C1 beschreibt eine wäßrige Reperfusionslösung zur Verminderung des Reperfusionsschadens nach akutem, peripherem Gefäßverschluß. Diese Reperfusionslösung soll unter vermindertem Reperfusionsdruck dem Patienten zugeführt werden, um den postischämischen Schaden zu vermindern.

Der Erfindung liegt die Aufgabe zugrunde, eine Reperfusionsvorrichtung der im Oberbegriff des Patentanspruchs 1 angegebenen Art zu schaffen, die eine Reperfusion mit einfachen Mitteln unter größter Sicherheit für den Patienten ermöglicht.

Die Lösung dieser Aufgabe erfolgt erfindungsgemäß mit den im kennzeichnenden Teil des Patentanspruchs 1 angegebenen Merkmalen.

Bei der erfindungsgemäßen Reperfusionsvorrichtung wird dem Patienten oxygeniertes Blut entzogen und dieses vom Patienten stammende Blut wird in einem Mischbehälter mit der Reperfusionslösung gemischt. Das Blut-Lösungs-Gemisch wird schließlich dem Patienten über einen Katheter wieder zugeführt, und zwar an einer Stelle, die stromauf von der behandelten Stenose liegt. Es wird also keine Blutkonserve benutzt, sondern arterielles Blut des Patienten, das dem Patienten nach Aufbereitung mit der Reperfusionslösung unmittelbar wieder zugeführt wird. Da das Blut nur kurze Zeit den Patientenkörper verläßt, werden Blutschädigungen weitgehend vermieden.

Die Blutdruckmessung erfolgt im Blutgefäß, so daß eine sehr genaue Blutdruckregelung möglich ist. Der Druck sollte etwa 50 mm Hg betragen. Wenn eine arterielle Blutdruckmessung nicht möglich ist, z.B. bei einer Behandlung der Herzkranzarterie, kann die Pumpe derart geregelt werden, daß sie eine konstante Durchflußmenge pro Zeiteinheit fördert. Diese Durchflußmenge beträgt etwa 50 ml/min. Eine solche Zuflußmenge erzeugt in einer Herzkranzarterie einen Druck von 50 mm Hg.

Die Blutentnahmevorrichtung kann an die zu behandelnde Arterie, stromauf von der Stenose, angeschlossen werden, jedoch ist es zweckmäßig, die Blutentnahme an einer anderen Arterie durchzuführen, weil dann eine kleinere Blutmenge in dem Mischbehälter gespeichert werden muß.

Bei Durchführung der Reperfusion wird zweckmäßigerweise das zu behandelnde Blutgefäß stromauf von der Stenose okkludiert und das Blut-Lösungs-Gemisch wird hinter der Okklusionsstelle in die Arterie geleitet. Zu diesem Zweck weist der Katheter einen Okklusionsballon auf und hinter diesem ist ein mit dem Flüssigkeitslumen des Katheters verbundener Auslaß angeordnet. Außerdem Flüssigkeitslumen ist am Katheter vorzugsweise ein Druckmeßlumen vorhanden, das an ein Druckmeßgerät angeschlossen ist.

Es sollte sichergestellt sein, daß in den extrakorporalen Blutkreislauf keine Luft eindringen kann, damit die Gefahr einer Luftembolie vermieden wird. Hierzu ist das extrakoroporale Schlauchsystem ohne lösbare Konnektoren ausgebildet. Im Falle lösbarer Konnektoren besteht die Gefahr des unbeabsichtigten Öffnens des Blutkreislaufs.

Ferner sollte sichergestellt sein, daß das dem Patienten zugeführte Gemisch extrakorporal auf Körpertemperatur gebracht oder gehalten wird. Hierzu ist an dem Mischbehälter eine geregelte Heizung vorgesehen. Ferner ist die extrakorporale Blutleitung, die vom Mischbehälter zum Patienten führt, mit einer Wärmeisolierung versehen.

Die Erfindung ermöglicht eine kontrollierte Reperfusion geschädigter Organe bzw. Extremitäten, wobei die Behandlungsdauer, während der das Blutgefäß von dem Blut-Lösungs-Gemisch mit geringem Druck bzw. geringer Strömungsrate durchströmt wird, etwa 30 Minuten betragen sollte. Danach ist das Gewebe so weit stabilisiert, daß die Reperfusion beendet werden kann, so daß nach Aufheben der Arterienokklusion das Blut mit dem vollen Blutdruck durch die Arterie strömen kann. Grundsätzlich reicht es aus, einen bestimmten Reperfusionsdruck, z.B. 50 mm Hg, für eine bestimmte Zeit von mehreren Minuten einzustellen, jedoch kann es auch zweckmäßig sein, die Reperfusion nach einem zeitabhängigen Druckprofil auszuführen, wobei der Reperfusionsdruck kontinuierlich oder stufenweise gesteigert wird. Diese Druck- bzw. Volumensteuerung kann von einem Rechner zeitabhängig durchgeführt werden.

Im folgenden werden unter Bezugnahme auf die Zeichnungen Ausführungsbeispiele der Erfindung näher erläutert.

Es zeigen:
- Fig. 1: eine schematische Darstellung des Anschlusses eines Reperfusators an den Patienten,
- Fig. 2: eine Darstellung einer einfachen Ausführungsform der Vorrichtung,
- Fig. 3: einen Abschnitt des in die zu behandelnde Arterie eingeführten Katheters und
- Fig. 4: einen Querschnitt durch den Katheter entlang der Linie IV-IV von Fig. 3.

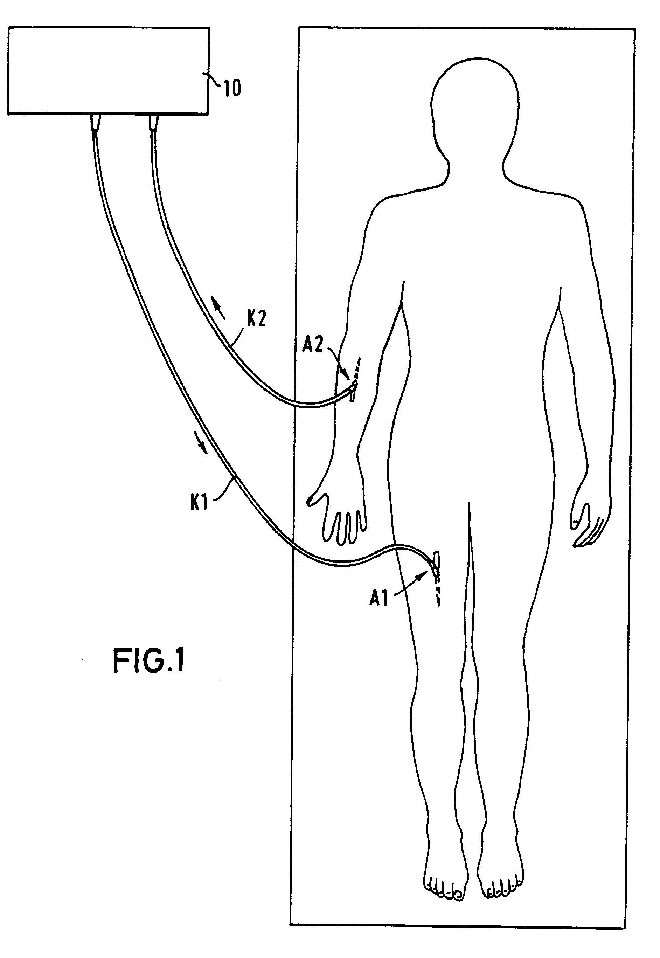

Gemäß Fig. 1 ist ein erster Katheter K1 in die zu behandelnde Arterie A1 verlegt. Der Katheter K1 wird von dem Reperfusator 10 mit Flüssigkeit versorgt, die in die Arterie A1 eingeführt wird.

Von einer anderen (gesunden) Arterie A2 des Patienten führt ein weiterer Katheter K2 zu dem Reperfusator 10. Aus der Arterie A2 strömt oxygeniertes Blut zum Reperfusator 10 wo dieses Blut nach einem vorbestimmten Mischungsverhältnis mit einer Reperfusionslösung gemischt wird, wie sie beispielsweise in DE 38 20 840 C1 beschrieben ist. Das Gemisch wird dann über den Katheter K1 zur behandelten Arterie A1 geführt.

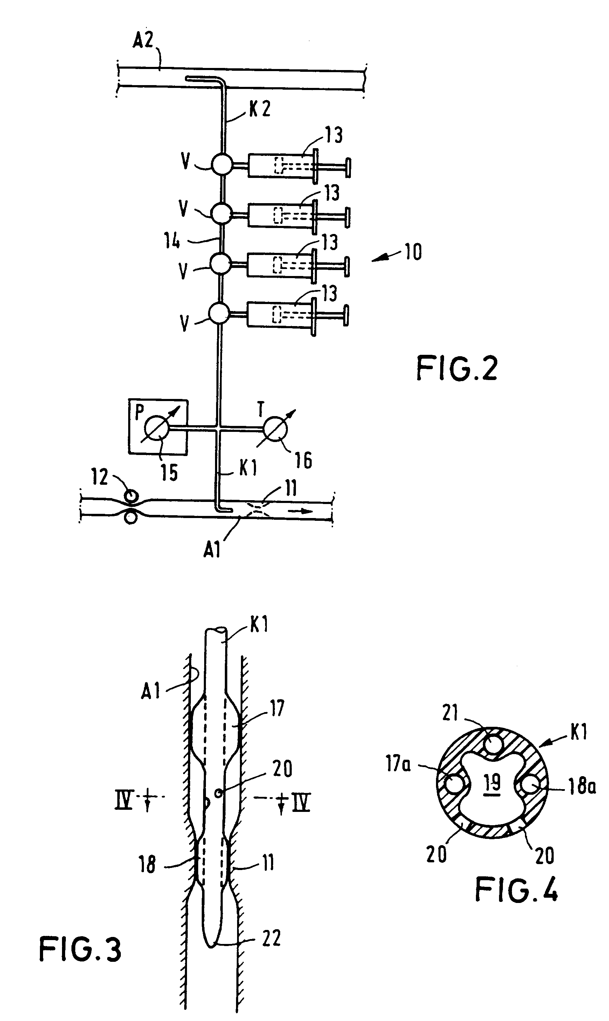

Fig. 2 zeigt eine sehr einfache Ausführungsform des Reperfusators 10, die für Handbetrieb geeignet ist, jedoch auch motorisch betrieben werden kann. Die kranke Arterie A1 wird stromauf von der Stenose 11 mit einer Klemme 12 abgeklemmt, die Stenose bzw. der Verschluß beseitigt und der Katheter K1 wird zwischen der ehemaligen Stenose 11 und der Klemme 12 verlegt.

Der gesunden Arterie A2 wird mittels eines einlumigen Katheters K2 Blut entnommen und in Spritzen 13 aufgezogen, die zum Teil mit flüssiger Reperfusionslösung gefüllt sind, so daß sich bei vollständiger Füllung der Spritzen 13 das gewünschte Blut-Lösungs-Gemisch einstellt. Die Spritzen 13 bilden in diesem Fall die Mischkammern. Diese Spritzen sind jeweils über ein Ventil V an eine Sammelleitung 14 angeschlossen, die mit dem Katheter K1 verbunden ist. Während des Füllvorgangs der Spritzen 13 sind die Ventile V so geschaltet, daß noch keine Flüssigkeit infundiert wird. Nach Abschluß des Füllvorganges kann die Reperfusion erfolgen, wobei die Ventile V so geschaltet werden, daß beim Ausdrücken einer Spritze 13 die Flüssigkeit aus dieser Spritze zum Katheter K1 strömt und vom Katheter K2 ferngehalten wird.

An die Sammelleitung 14 sind ein Druckmesser 15 und ein Temperaturmesser 16 angeschlossen, die jeweils ein Alarmsignal erzeugen, wenn der zu messenden Parameter außerhalb eines vorgegebenen Zulässigkeitsbereichs liegt.

Nachdem sämtliche Spritzen aufgezogen worden sind, werden die Ventile V umgeschaltet und der Inhalt der Spritzen wird durch den Arzt kontrolliert ausgedrückt, so daß über den Katheter K1 eine kontrollierte Reperfusion erfolgt. Dieser Vorgang wird nach etwa 30 Minuten abgeschlossen und dann wird der Katheter K1 entfernt und die Klemme 12 geöffnet.

Fign. 3 und 4 zeigen eine Ausführungsform des Katheters K1. Dieser Katheter besteht aus einem flexiblen Schlauch, der zwei Ballons 17 und 18 mit gegenseitigem axialen Abstand aufweist. Der Ballon 17 ist ein Okklusionsballon zum Verschließen der Arterie A1 stromauf von der Stenose 11 und der Ballon 18 ist ein Dilatationsballon zum Aufweiten des Gefäßes A1 an der Stenose 11. Das Flüssigkeitslumen 19 des Katheters ist mit Auslässen 20 versehen, die zwischen den beiden Ballons 17 und 18 angeordnet sind. Ein Lumen 17a des Katheters K1 dient zum Zuführen von Luft in den Ballon 17 und ein Lumen 18a dient zum Zuführen von Luft in den Ballon 18. Ein weiteres Lumen 21 dient der Druckmessung und beispielsweise zum Anschluß des Druckmessers 15 in Fig. 2. Das Lumen 21 tritt vorzugsweise an der Katheterspitze 22 aus.

## Patentansprüche

1. Reperfusionsvorrichtung für die Reperfusion von Blutgefäßen, mit einem in das Blutgefäß einführbaren Katheter (K1), mit einer Blutentnahmevorrichtung (K2, 13) zur Entnahme von oxygeniertem arteriellem Blut von einem Patienten, mit einer Quelle für Reperfusionslösung, mit einem Mischelement zur Bildung eines Gemisches aus dem vom Patienten entnommenen oxygenierten arteriellen Blut und der Reperfusionslösung, und mit einer Fördervorrichtung, welche das Blut-Lösungs-Gemisch in den Katheter (K1) treibt,
**dadurch gekennzeichnet,**
daß die Fördervorrichtung eine Pumpvorrichtung ist und in Abhängigkeit vom Druck im behandelten Blutgefäß (A1) geregelt ist, und daß der Katheter (K1) an der Katheterspitze (22) ein Druckmeßlumen (21) oder eine Drucksonde aufweist.

2. Reperfusionsvorrichtung nach Anspruch 1,
**dadurch gekennzeichnet,**
daß ein zweiter Katheter (K2) in der Blutentnahmevorrichtung vorgesehen ist, der zur Blutentnahme in ein anderes Blutgefäß (A2) einführbar ist.

3. Reperfusionsvorrichtung nach Anspruch 1 oder 2,
**dadurch gekennzeichnet,**
daß der Katheter (K1) im Abstand voneinander einen Dilatationsballon (18) und einen Okklusionsballon (17) aufweist und daß zwischen diesen Ballons ein mit dem Flüssigkeitslumen (19) des Katheters verbundener Auslaß (20) angeordnet ist.

4. Reperfusionsvorrichtung nach einem der Ansprüche 1 bis 3,
**dadurch gekennzeichnet,**
daß das Mischelement (13) temperaturgeregelt beheizt ist.

5. Reperfusionsvorrichtung nach einem der Ansprüche 1 bis 4,
**dadurch gekennzeichnet,**
daß der Katheter (K1) mit dem Mischelement fest und ohne lösbare Konnektoren verbunden ist.

6. Reperfusionsvorrichtung nach einem der Ansprüche 1 bis 5,
**dadurch gekennzeichnet,**
daß das vom Mischelement zum Patienten führende extrakorporale Teil des Katheters (K1) wärmeisoliert ist.

## Claims

1. A reperfusion device for the reperfusion of blood vessels, having a catheter (K1) which can be introduced into the blood vessel, having a blood-collecting device (K2, 13) for taking oxygenated arterial blood from a patient, having a source for reperfusion solution, having a mixing element for forming a mixture from the oxygenated arterial blood taken from the patient and the reperfusion solution, and having a conveying device, which drives the blood-solution mixture into the catheter (K1),
**characterized in that** the conveying device is a pumping device and is controlled in dependence on the pressure in the treated blood vessel (A1),
**and in that** the catheter (K1) comprises a pressure measuring lumen (21) or a probe at the catheter point (22).

2. A reperfusion device according to Claim 1,
**characterized in that** a second catheter (K2) is provided in the blood-collecting device and can be introduced into another blood vessel (A2) for taking blood.

3. A reperfusion device according to Claim 1 or 2,
**characterized in that** the catheter (K1) comprises a dilation balloon (18) and an occlusion balloon (17) spaced from one another,
**and that** between these balloons is disposed an outlet (20) connected to the fluid lumen (19) of the catheter.

4. A reperfusion device according to one of Claims 1 to 3,
**characterized in that** the mixing element (13) is heated under temperature control.

5. A reperfusion device according to one of Claims 1 to 4,
**characterised in that** the catheter (K1) is connected to the mixing element in a secure manner and without detachable connectors.

6. A reperfusion device according to one of Claims 1 to 5,
**characterised in that** the extracorporal part of the catheter (K1) leading from the mixing element to the patient is heat-insulated.

## Revendications

1. Dispositif de reperfusion pour reperfuser des vaisseaux sanguins, comprenant un cathéter (K1) pouvant être introduit dans le vaisseau sanguin, un dispositif de prélèvement du sang (K2, 13) pour prélever du sang artériel oxygéné d'un patient, une source pour une solution de reperfusion, un élément de mélange pour former un mélange à partir du sang artériel oxygéné prélevé sur le patient et de la solution de reperfusion, et un dispositif d'alimentation qui amène le mélange sang'solution dans le cathéter (K1), caractérisé en ce que le dispositif d'alimentation est une pompe et est réglé en fonction de la pression dans le vaisseau sanguin traité (A1), et en ce que le cathéter (K1) présente à la pointe du cathéter (22) un canal pour mesurer la pression (21) ou une sonde manométrique.

2. Dispositif de reperfusion selon la revendication 1, caractérisé en ce que dans le dispositif de prélèvement du sang est prévu un deuxième cathéter (K2) qui peut être introduit dans un autre vaisseau sanguin (A2) pour prélever du sang.

3. Dispositif de reperfusion selon la revendication 1 ou 2, caractérisé en ce que le cathéter (K1) présente un ballon de dilatation (18) et un ballon d'occlusion (17) espacés l'un de l'autre, et en ce qu'entre ces ballons se situe un orifice de sortie (20) relié au canal de liquide (19) du cathéter.

4. Dispositif de reperfusion selon l'une des revendications 1 à 3, caractérisé en ce que l'élément mélangeur (13) est chauffé sous contrôle de la température.

5. Dispositif de reperfusion selon l'une des revendications 1 à 4, caractérisé en ce que le cathéter (K1) est relié fixement à l'élément mélangeur sans connecteurs amovibles.

6. Dispositif de reperfusion selon l'une des revendications 1 à 5, caractérisé en ce que la partie extracorporelle du cathéter (K1) allant de l'élément de mélange au patient est calorifugée.
